# EUROPEAN PATENT APPLICATION

(11) **EP 2 020 387 A1**
(43) Date of publication of application: **04.02.2009**
(21) Application number: 07120766.6
(22) Date of filing: 15.11.2007
(51) Int. Cl.: B65D 85/50, A01K 63/00

(54) **Firefly Container**

(30) Priority: 31.07.2007 US 831219
(71) Applicant: Taikong Corp., 115 Taipei (TW)
(72) Inventor: Lin, Shiue-Lian, 115, Taipei (TW); Lee, Chih-Fei, 115, Taipei (TW)
(74) Representative: Lermer, Christoph

(57) **Abstract**

The present invention provides a firefly container (100), comprising transparent outer walls (110) and air holes. After putting nutrients, water, shelters, and matrixes into the viewer, fireflies (180) can be allowed to live in it. Moreover, if a handling device (140) is installed on the present invention, it can be used as a "firefly lantern" and provides edutainmental functions.

## Description

### BACKGROUND OF THE INVENTION

### (a) Technical Field of the Invention

The present invention relates to firefly-container, and in particular to a firefly container for use in observing and feeding fireflies, which comprises transparent walls and an observer is able to observe metamorphosis of fireflies through the transparent walls.

### (b) Brief Description of the Prior Art

Children as well as the adults are fascinated by fireflies as they fluorescence. If fireflies are used as teaching material for the subject of biology, the degree of acceptance in rearing insects will increase and the public will understand the life cycle of insects. However, fireflies are forced to be reared in an ordinary aquarium or insect container because there was no container specifically designed for feeding and observing fireflies available in the market nowadays. These aquariums and insect containers are usually huge and heavy that they can only be displayed at a fixed position. People can merely watch the insects at a specific position, and these boxes are not conveniently to be moved, or the aquariums and insect boxes cannot be carried along. In addition, the shapes of these feeding containers available in the market are mostly cuboid and cubic shape, which are not attracted to consumers.

In order to improve the designs of aquariums and insect boxes available in the market, the present invention integrates the fluorescent characteristic of fireflies into the design of insect container, so as to enhance the will of the public to contact and rear fireflies.

### SUMMARY OF THE INVENTION

The present invention provides a firefly-container for observing and feeding of fireflies, which comprises transparent walls having at least one hole for ventilation and providing a habitat for fireflies by supplying nutrients and water, covering object and a feeding base, to allow the fireflies to live. The container is provided with a component for hand-carrying such that the container is used as a firefly-lantern, and this provides teaching and funs to children.

### BRIEF DESCRIPTION OF THE DRAWINGS

Figure 1 shows the structure of a firefly-container in accordance with the present invention.
Figure 2 shows the actual usage of the firefly-container in accordance with the present invention.

### DETAILED DESCRIPTION OF THE PREFERRED EMBODIMENT

The present invention provides a firefly-container for observing and feeding of fireflies, which comprises transparent walls having at least one hole for ventilation and providing a habitat for fireflies by supplying nutrients, water, covers and the feeding bases, to allow the fireflies to live. The container is provided with a component for hand-carrying such that the container is used as a firefly-lantern, and this provides teaching and funs to children.

The walls of the firefly-container of present invention are made from transparent materials, such as glass and plastic et al. to allow users to observe the metamorphosis of fireflies conveniently and to allow fluorescence emitted by fireflies be seen easily.

The firefly-container of the present invention also provides air-hole for ventilation between inside and outside of the container to avoid fireflies from suffocating within the container. The air-hole can be openings on the walls or nets which are used to replace one of the walls, such as the top end of the container. The air-holes are so small that fireflies in the firefly-container will not escape to the outside.

A feeding base placed in the firefly-container will discharge water and to maintain humidity in the firefly-container, so as to provide an inhabitation for fireflies. Furthermore, if plants are grown in the firefly-container, the feeding base acts as a ground for plants growing. The feeding base composed of inorganic soil, organic soil or artificial substrates. The definition of inorganic soil is products effloresced from rocks and mines in nature, such as earth, gravel and sand; the definition of organic soil is products which are rotten and decomposed from organism, such as dead woods, dead leaves and rotten woods; and the artificial substrates include paper-products, plastics, sponge or other artificial pad material for insect-feeding.

The covering objects within the container are used to shelter fireflies from lights, and the covering objects also provide a place of crawling and resting. For this reason, any object which can block lights is used as a covering object, such as plants, laminated board, and miniature structure and housing models.

A damp environment is required for the growth of fireflies and the environment is maintained by placing a water pool in the firefly-container or sprinkling water regularly. The firefly-container of the present invention further comprises water treatment equipment or a water conditioner to keep the pool water quality constant.

The firefly-container of the present invention further comprises a handle for convenient moving of the firefly-container. All decorative components in the firefly-container are firmly secured so as to avoid the living environment of fireflies from being destroyed when the firefly-container is being moved from place to place.

The firefly-container of the present invention provides a living environment for each developmental stage of fireflies, which include egg, larva, pupa and imago. Therefore, it is easier to feed fireflies and observe the life cycle of firefly using the firefly-container of the present invention.

The firefly-container of the present invention provides fun and teaching to children and the knowledge of life cycle of fireflies. In addition, the firefly-container is being shown as a work of art because of its aesthetic appearance of various shapes. When a hand-carrying component is attached, the container is turned into a "firefly-lantern".

### Examples

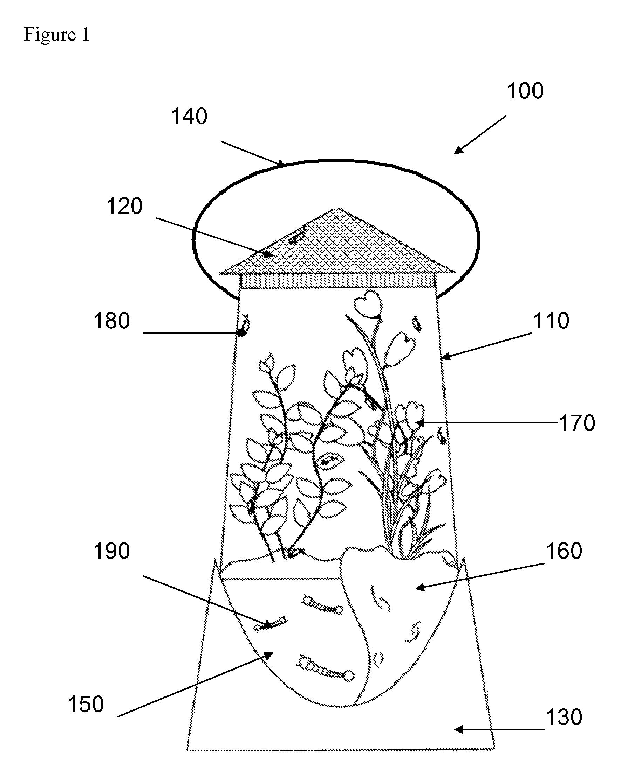

The structure of a firefly-container of the present invention was shown in FIG.1. A major part of the present firefly-container included transparent walls, having air-holes for ventilation. The transparent walls of the firefly-container were made of hyaline glass, hyaline plastics or other transparent materials. The air-holes of the present firefly-container were displayed in form of openings around the peripheral walls or a nets structure.

To furnish ornamental or decorative objects inside the firefly-container conveniently, the present invention comprised a movable sliding door or a lid. Detachable walls for the firefly-container were also designed for the same purpose. Besides, the walls could be detached for cleaning and feeding the fireflies in the container. In one embodiment, the air-hole and detachable walls of firefly-container were designed to be an integer. There was a handle attached to the container and the size and weight of the container allowed it to be easy carried in one hand.

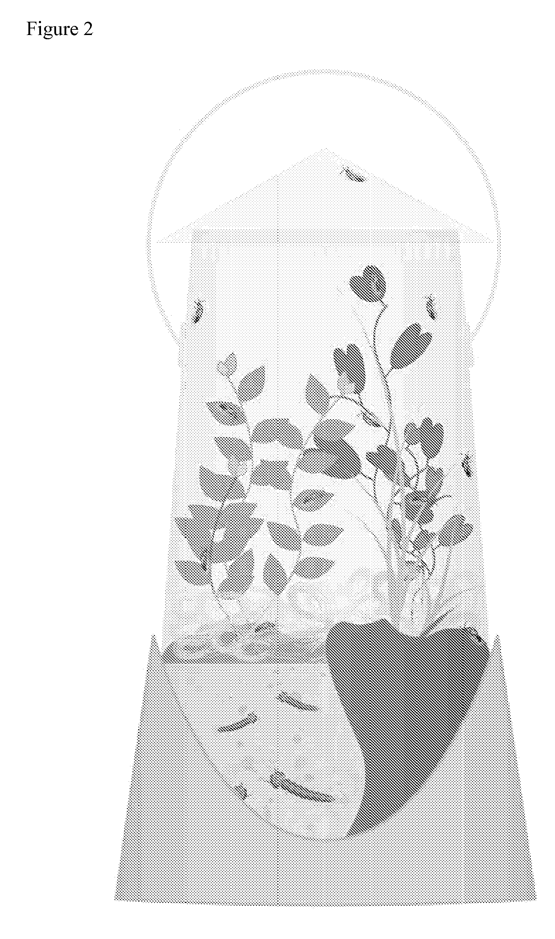

Figure 2 was a schematic view showing the feeding of fireflies in the firefly-container of the present invention. The present invention of firefly-container, which comprises a pool, soil and plants et al., can provide a favorable environment for the development of fireflies. Additionally, the fluorescence of fireflies was easily emitted to outside because of the transparent walls of the firefly-container, which made the present firefly-container to be a firefly-lantern.

While the invention has been described with respect to preferred embodiment, it will be clear to those skilled in the art that modifications and improvements may be made to the invention without departing from the spirit and scope of the invention. Therefore, the invention is not to be limited by the specific illustrative embodiment, but only by the scope of the appended claims.

## Claims

1. A firefly-container for observing and feeding fireflies, comprising a plurality of transparent walls having at least one air-hole for ventilation providing an appropriate environment for the development of fireflies by placing required nutrients and water, and the container allows each phase of metamorphosis of the fireflies to be seen through the transparent walls.

2. The firefly-container of claim 1, wherein the transparent walls are made of hyaline glass or hyaline plastics.

3. The firefly-container of claim 1, wherein the air-hole for ventilation between inside and outside firefly-container is in the form of openings on walls, net walls or the like which allow ventilation.

4. The firefly-container of claim 1, wherein the environment includes a feeding base, a covering object and a damp condition.

5. The firefly-container of claim 4, wherein the feeding base is composed of inorganic soil, organic soil or artificial substrates, which discharge water and maintain humidity of the firefly-container, so as to provide an inhabitation environment for fireflies.

6. The firefly-container of claim 5, wherein the inorganic soil is one selected from the group consisting of earth, gravel, sand or products effloresced from rocks and mines in nature; wherein the organic soil is one selected from the group consisting of dead woods, dead leaves, rotten woods or product rotten and decomposed from organism; wherein the artificial substrates include paper-products, plastics, sponge or other artificial pad material for insects-feeding.

7. The firefly-container of claim 4, wherein the covering object is a plant, a board, a cave or other objects, which can shelter fireflies from external lights.

8. The firefly-container of claim 4, wherein the damp condition is created by having a pool or a wet feeding base in the firefly-container.

9. The firefly-container of claim 8 further comprises a water treatment equipment or a water conditioner to keep the pool water quality constant.

10. The firefly-container of claim 1, wherein the metamorphosis of fireflies are egg phase, larva phase, pupa phase or imago phase.

11. The firefly-container of claim 1 further comprises a hand-carrying component.

12. The firefly-container of claim 1, wherein the ornamental design of the firefly container is cuboid or of any shape.
